# EUROPEAN PATENT APPLICATION

(11) **EP 2 050 404 A1**
(43) Date of publication of application: **22.04.2009**
(21) Application number: 08253353.0
(22) Date of filing: 16.10.2008
(51) Int. Cl.: A61B 17/34, A61B 17/00

(54) **Anchoring Cannula**

(30) Priority: 17.10.2007 US 980579; 08.10.2008 US 247259
(71) Applicant: Tyco Healthcare Group LP, North Haven, CT 06473 (US)
(72) Inventor: Bettuchi, Michael, Middletown, CT 06457 (US); Heinrich, Russell, Madisnon, CT 06443 (US); Sung, Jason, North Branford, CT 06471 (US)
(74) Representative: Soames, Candida Jane

(57) **Abstract**

The present disclosure provides cannulas for use in surgery. The cannulas possess an anchoring member at a portion thereof which adopts an alternate shape upon the application of energy, thereby securing the cannula to tissue.

## Description

### CROSS-REFERENCE TO RELATED APPLICATIONS

This application claims the benefit of and priority to U.S. Provisional Patent Application No. 60/980,579, filed October 17, 2007, the entire disclosure of which is incorporated by reference herein.

### TECHNICAL FIELD

The present disclosure provides cannulas for use in surgical procedures and, more particularly, to an anchoring cannula possessing a material on or as part of a portion thereof which alters its configuration upon the application of energy to anchor the cannula to tissue upon insertion in a patient's body. Methods of using such a cannula are also provided.

### BACKGROUND

During laparoscopic procedures, cannulas are utilized to provide an access port for surgical instruments and a conduit for introducing insufflation gases into the body cavity. In embodiments, a sharp trocar may be positioned within the cannula and utilized to puncture or pierce the tissue or abdominal wall. Thereafter the trocar may be removed, leaving the cannula in place and insufflation gases forced into the body cavity to form an anatomical operating space. Retention of access ports such as cannulas during a laparoscopic procedure is very important, as the ports can be accidentally ejected from the patient, resulting in inconvenience to the surgeon, loss of pneumoperitoneum, and increased procedure time.

In order to prevent the cannula from migrating in or out through the incision, some cannulas may be provided with anchoring structures to prevent the cannula from slipping out of the incision. For example, balloons have been used in some devices to assist in anchoring a cannula, as disclosed in U.S. Patent No. 5,468,248 and U.S. Patent Application Publication No. 2004/0138702, the entire disclosures of each of which are incorporated by reference herein. However, unless the anchoring structure is firmly secured against the tissue, leakage of insufflation gases may occur. Thus, means for anchoring cannulas to secure the cannula to the tissue and prevent leakage of insufflation gases remain desirable.

### SUMMARY

The present disclosure provides cannulas for use in surgery. In embodiments, the cannulas may possess an anchoring member at or on a portion thereof. The anchoring member may alter its shape upon the application of energy, thereby securing the cannula to tissue.

In embodiments, a cannula of the present disclosure may include a cannula body possessing a longitudinally extending central working passage and at least one anchoring member. The anchoring member may include shape memory polymers, shape memory metals, shape memory alloys, electroactive polymers, and combinations thereof. The anchoring members may be secured to the shaft using chemical methods, physical methods, and combinations thereof.

In embodiments, the anchoring member of a cannula of the present disclosure may have a permanent shape including a ring with an annular diameter, sometimes referred to as an expanded annular diameter, and a temporary shape with a smaller compressed annular diameter. In other embodiments, the anchoring member may have a permanent shape including a ring with a compressed annular diameter, and a temporary shape with a larger expanded annular diameter.

In other embodiments, the anchoring member of a cannula of the present disclosure may have a permanent shape including angular protrusions and a temporary shape including a smooth, flat configuration.

In some embodiments, an anchoring member of a cannula of the present disclosure may also include a medicinal agent.

Methods for utilizing cannulas of the present disclosure are also provided.

### BRIEF DESCRIPTION OF THE DRAWINGS

FIGS. 1A and 1B illustrate a cannula which possesses at least one anchoring member that may be selectively expandable at one or more selected longitudinal locations;

FIGS. 2A, 2B, 2C, 2D, 2E and 2F illustrate a cannula of the present disclosure possessing anchoring members which cause the cannula to form an accordion configuration upon the application of energy;

FIGS. 3A and 3B illustrate a cannula of the present disclosure possessing an anchoring member with gaps therein, the anchoring member forming V shape protrusions upon application of suitable energy; and

FIGS. 4A and 4B illustrate an alternate cannula of the present disclosure possessing an anchoring member applied to a cannula with gaps therein whereby the anchoring member forms protrusions and adopts a frustoconical configuration upon application of suitable energy.

### DETAILED DESCRIPTION

The present disclosure provides an anchoring cannula including a cannula body having an anchoring member affixed thereto or incorporated therein. The anchoring member may be made of a material which can change its shape upon the application of energy such as electricity or heat, including body heat, to secure a cannula within the body. Such materials include, for example, shape memory polymers, shape memory metals, shape memory alloys, electroactive polymers, combinations thereof, and the like.

In embodiments, shape memory materials may be utilized to form the anchoring member on a cannula of the present disclosure. Such shape memory materials possess a permanent shape and a temporary shape. In embodiments, the temporary shape is of a configuration which enhances the ability of one to introduce a cannula possessing an anchoring member into a patient's body, while the permanent shape is of a configuration which enhances the retention of the cannula at the site of an incision. Suitable shape memory polymeric materials which may be utilized to fashion an anchoring member include, for example, polyurethanes, poly(styrene-butadiene) block copolymers, polynorbomenes, caprolactones, dioxanones, diol esters including oligo (epsilon caprolactone) diol, lactic acid, lactide, glycolic acid, glycolide, ether-ester diols including oligo (p-dioxanone) diol, carbonates including trimethylene carbonate, combinations thereof, and the like. In embodiments, the shape memory polymer may be a copolymer of two components with different thermal characteristics, such as oligo (epsilon-caprolactone) dimethacrylates and butyl acrylates including poly(epsilon-caprolactone) dimethacrylate-poly (n-butyl acrylate), or a diol ester and an ether-ester diol such as oligo (epsilon caprolactone) diol/oligo (p-dioxanone) diol copolymers. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory Polymers-Biodegradable Sutures," Materials World, Vol. 10, no. 7, pp. 29-30 (July 2002), the entire disclosure of which is incorporated by reference herein.

In other embodiments, blends of materials may be utilized as the shape memory polymeric material including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

Other examples of these shape memory polymers and methods for forming permanent and temporary shapes therewith are set forth in Lendlein et al., "Shape memory polymers as stimuli-sensitive implant materials," Clinical Hemorheology and Microcirculation, 32 (2005) 105-116, and Lendlein et al., "Biodegradable, Elastic Shape-Memory Polymers for Potential Biomedical Applications," Science, Vol. 269 (2002) 1673-1676, the entire disclosures of each of which are incorporated by reference herein.

In embodiments, the anchoring member of a cannula of the present disclosure may have a permanent shape including a ring with a first annular diameter, sometimes referred to herein as an expanded annular diameter, and a temporary shape with a smaller circumference, sometimes referred to as a compressed annular diameter. In other embodiments, the anchoring member may have a permanent shape including a ring with a first circumference, which may be a compressed annular diameter, and a temporary shape with a larger circumference, which may be referred to as an expanded annular diameter.

For example, the shape memory polymeric materials of the present disclosure may, in embodiments, be compressed or expanded into temporary forms up to about four times larger in diameter or four times smaller in diameter than their permanent shape. Thus, in embodiments, shape polymeric materials may be fashioned into an anchoring member having a permanent shape that possesses an expanded diameter up to about four times greater than its thinner compressed temporary diameter. In other embodiments, shape polymeric materials may be utilized to fashion an anchoring member having a permanent shape that possesses a compressed diameter up to about four times smaller than its thicker, expanded, temporary diameter. In yet other embodiments, combinations of shape memory polymeric materials having both expanded and compressed permanent shapes may be utilized to form an anchoring member on or within the surface of a cannula.

The anchoring members thus prepared recover their originally memorized shape on heating, either by placement in a patient's body or the addition of exogenous heat at a prescribed temperature, in embodiments above Tₜᵣₐₙₛ for the shape memory polymer utilized. In embodiments, the shape polymeric material utilized to anchor a cannula in an incision may have a temporary shape possessing a thickness, referred to in embodiments as a compressed annular diameter, of from about 0.002 inches to about 0.01 inches, in embodiments from about 0.005 inches to about 0.008 inches, and expand to an annular diameter of from about 0.75 inches to about 1.75 inches, in embodiments from about 1 inch to about 1.5 inches, referred to herein as its permanent shape or its expanded annular diameter, to anchor a cannula at the opening of an incision.

In other embodiments, the shape memory polymeric material utilized to anchor a cannula in an incision may have a temporary shape possessing a thickness, in embodiments as noted above which may be referred to as an expanded annular diameter, of from about 0.75 inches to about 1.75 inches, in embodiments from about 1 inch to about 1.5 inches, and compress, retract, or contract to its permanent shape, referred to in embodiments as a compressed annular diameter, of from about 0.002 inches to about 0.01 inches, in embodiments from about 0.005 inches to about 0.008 inches. Such an embodiment may be useful to facilitate removal of a cannula after insertion at the site of an incision.

Similarly, shape memory polymeric materials may be configured so that they possess a smooth, flat temporary shape which facilitates insertion of a cannula possessing an anchoring member fashioned with such a material into tissue. In other embodiments, shape memory polymeric materials may be configured so that they may possess a temporary shape which may be arcuate to permit placement of the anchoring member formed of shape memory polymeric materials in or around all or part of a cannula. Such materials may possess angular protrusions or other similar configurations as their permanent shape, which they will adopt upon the application of suitable energy including electricity or heat, including body heat. The permanent angular or similar configuration which the material utilized to form the anchoring member adopts may assist in anchoring the cannula of the present disclosure at the tissue located at the opening of an incision.

In embodiments, a molding process may be utilized to produce the anchoring member of the present disclosure. Plastic molding methods may be employed and may include, but are not limited to, melt molding, solution molding and the like. Injection molding, extrusion molding, compression molding and other methods can also be used as the melt molding technique. Once placed in the mold with the proper configuration and dimensions, the anchoring member may be heated to a suitable temperature, in embodiments at a temperature referred to as the permanent temperature (Tₚₑᵣₘ) which may, in embodiments, be the melting temperature of the shape memory polymeric material utilized to form the anchoring member. Heating of the anchoring member may be at suitable temperatures, for example, from about 40° C to about 180° C, in embodiments from about 45° C to about 60° C, for a period of time from about 10 minutes to about 60 minutes, in embodiments from about 15 minutes to about 20 minutes, to obtain the permanent shape and dimensions of the anchoring member, including its desired configuration and thickness.

After the anchoring member with the desired configuration and thickness has been formed, the anchoring member may be deformed at a deforming temperature to obtain a thinner temporary shape, a temporary shape having an alternate configuration, or both. In other embodiments, the anchoring member may be deformed at a deforming temperature to obtain a thicker temporary shape, a temporary shape having an alternate configuration, or both. There are no particular limitations on the manner in which the deformation can be achieved. Deformation can be achieved either by hand or by way of a suitable device selected to provide the desired thickness and/or configuration to the anchoring member.

Suitable temperatures for deformation will vary depending on the shape memory polymer utilized, but generally may be above the transition temperature of the polymer (Tₜᵣₐₙₛ), but below the Tₚₑᵣₘ. In embodiments, the shape memory polymer may be cooled from its Tₚₑᵣₘ to a lower temperature which remains above the Tₜᵣₐₙₛ and deformed, in embodiments by compression, to a thinner temporary shape. As noted above, in other embodiments the shape memory polymer may be cooled from its Tₚₑᵣₘ to a lower temperature which remains above the Tₜᵣₐₙₛ and deformed, in embodiments by expansion, to a thicker temporary shape. As also noted above, in other embodiments the shape memory polymer may be cooled from its Tₚₑᵣₘ to a lower temperature which remains above the Tₜᵣₐₙₛ and deformed, in embodiments by straightening, so that the permanent shape includes angular protrusions and the temporary shape is flat and smooth.

The temperature for deformation treatment of the anchoring member molded with a previously memorized shape is one that makes possible ready deformation without producing cracks and should not exceed the temperature adopted for the shape memorization (e.g., Tₚₑᵣₘ). Deformation treatment at a temperature exceeding that for the original shape memorization may cause the object to memorize a new deformed shape.

In other embodiments, the anchoring member may be configured to its temporary shape and cooled to room temperature (about 20° C to about 25° C) to obtain its temporary shape, although the temperature may differ depending upon the particular polymer employed. The anchoring member may then be cooled to a temperature below Tₜᵣₐₙₛ, at which time the anchoring member of the present disclosure may be affixed to, or utilized in the manufacture of, the anchoring cannula as described above and is ready for use. As the Tₜᵣₐₙₛ is usually greater than room temperature, in embodiments cooling to room temperature may be sufficient to form the temporary shape.

The anchoring member may be deformed to its temporary shape prior to its attachment to the cannula or, in other embodiments, the anchoring member may be deformed to its temporary shape after attachment to the cannula. An anchoring member may be secured to a cannula in any way, including chemically and/or physically such as by adhesives, sealants, glues, and the like or, in some embodiments, the use of locking rings located on the proximal and distal sides of an anchoring member. In other embodiments, the anchoring member may be compressed to a dimension fitting within a recess or shallow depression on the surface of the cannula. As noted above, in other embodiments, the shape memory material may be utilized to form the cannula itself.

The anchoring members thus prepared recover their originally memorized permanent shapes on heating, either by placement in a patient's body or the addition of exogenous heat at a prescribed temperature, in embodiments above Tₜᵣₐₙₛ for the shape memory polymer utilized.

Other shape memory materials, including shape memory metals and metal alloys such as Nitinol, may be used to form the anchoring member.

In order to keep the shape and thickness of the anchoring member in its temporary shape, the shape-memory anchoring member of the present disclosure may be stored at a temperature which will not cause plastic deformation of the polymers or premature switching to the permanent shape. In embodiments, the shape-memory anchoring member may be stored in a refrigerator.

As the anchoring members of the present disclosure are utilized in a living body, heating with body heat (about 37° C) is possible. In such a case, the temperature for shape memorization may be as low as possible. In embodiments, recovery of the permanent shape may occur from about 1 second to about 5 seconds after insertion into tissue.

However, in some embodiments a higher shape-memory temperature may be desirable in order to make the shape recover at a slightly higher temperature than body temperature. Thus, in some cases releasing the anchoring member from deformation to recover the originally memorized permanent shape can be achieved by heating. On heating at a temperature from about 30° C to about 50° C, in embodiments from about 39° C to about 43° C, the temporary shape may be released and the memorized permanent shape recovered. The higher the temperature for heating, the shorter the time for recovery of the originally memorized shape. The method for this heating is not limited. Heating can be accomplished by using a gas or liquid heating medium, heating devices, ultrasonic waves, or the like. Of course, in an application involving a living body, care may be taken to utilize a heating temperature which will not cause burns. When a liquid heating medium is used, physiological saline solution may be desirable.

Upon insertion into the incision, the anchoring members made of a shape memory material may adopt their permanent configurations to assist in forming the anchoring configuration capable of anchoring a cannula at the site of incision, either by the heat of the patient, the application of heat from an exogenous source. In embodiments, where a shape memory polymer is utilized, the heat of the body (about 37° C), may be sufficient for the anchoring members to form their permanent anchoring shape. In other embodiments, heat may be applied to the anchoring member, in embodiments from about 39° C to about 43° C (just above human body temperature), to enhance the return of the shape memory polymer to its permanent anchoring shape.

Similarly, in other embodiments electrically active polymers, also known as electroactive polymers, which can alter their configuration upon application of electricity, may be utilized to fashion an anchoring member to secure a cannula within the body. Suitable examples of electroactive polymers include poly(aniline), substituted poly(aniline)s, polycarbazoles, substituted polycarbazoles, polyindoles, poly(pyrrole)s, substituted poly(pyrrole)s, poly(thiophene)s, substituted poly(thiophene)s, poly(acetylene)s, poly(ethylene dioxythiophene)s, poly(ethylenedioxypyrrole)s, poly(p-phenylene vinylene)s, and the like, or combinations including at least one of the foregoing electroactive polymers. Blends or copolymers or composites of the foregoing electroactive polymers may also be used.

Similar to the change in shape which a shape memory material may undergo upon the application of energy, such as heat, in embodiments an electroactive polymer may undergo a change in shape upon the application of electricity from a low voltage electrical source (such as a battery). Electricity may also be utilized, in embodiments, to promote the change in shape of a shape memory alloy such as Nitinol. Suitable amounts of electricity which may be applied to effect such change will vary with the electroactive polymer or shape memory alloy utilized, but can be from about 5 volts to about 30 volts, in embodiments from about 10 volts to about 20 volts. The application will result in the anchoring member constructed of the electroactive polymer to change its shape to an anchoring structure capable of anchoring a cannula at the site of an incision.

While an electroactive polymer does not have the same permanent shape and temporary shape as those terms are described above with respect to shape memory polymers, as used herein the term "permanent shape" as applied to an electroactive polymer may refer to, in embodiments, the shape the electroactive polymer adopts upon the application of electricity, and the term "temporary shape" as applied to an electroactive polymer may refer to, in embodiments, the shape the electroactive polymer adopts in the absence of electricity.

There is also disclosed a method of securing a cannula to tissue which includes providing the disclosed cannula. The cannula is inserted through an incision in the tissue to position the anchoring member in the opening, and the anchoring member is activated by the application of heat, such as body heat, or electricity to assist in the retention of the cannula during a laparoscopic procedure. While a shape memory polymer may return to its temporary shape upon removal of heat, in embodiments the shape memory polymer may remain in an expanded state. Where an electroactive polymer or a shape memory alloy such as Nitinol is used to form the anchoring member, the polymer or Nitinol could return to its original shape once the energy source is disconnected, thereby requiring a much lower withdrawal force to remove the cannula from a patient's body.

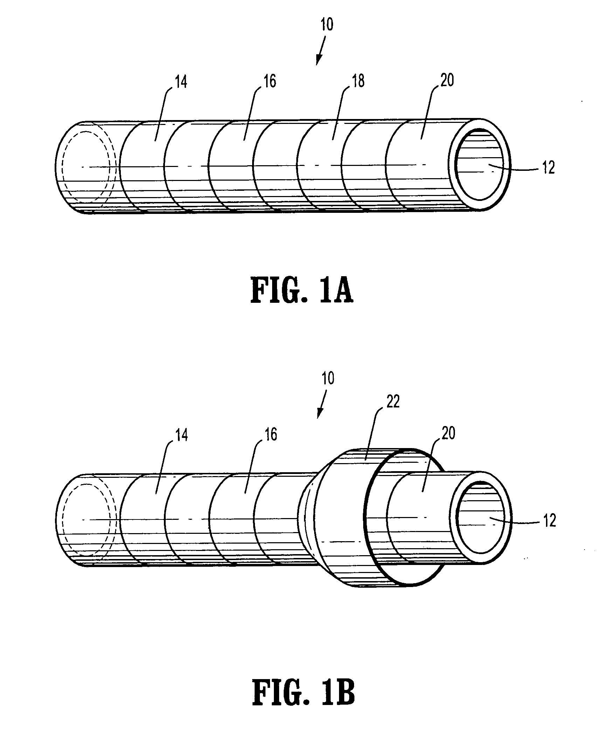

FIG. 1 A illustrates a cannula 10 which possesses an anchoring member at one or more selected longitudinal locations. Cannula 10 may be formed of any material. The cannula 10 possesses a longitudinally extending central working passage 12 and may, in embodiments, include a series of anchoring members. The anchoring members illustrated include a segment 14, a segment 16, a segment 18, and a segment 20. As an example, segments 14, 16, 18, and 20 may be expandable upon the application of heat where it is formed of a shape memory polymer, or upon the application of electricity from a low voltage electrical source (such as a battery), where the expandable anchoring member is formed of an electroactive polymer or a shape memory alloy such as Nitinol. In other words, the expandable segments illustrated as segments 14, 16, 18, and 20 have a permanent shape like a ring with a first circumference and a temporary shape with a smaller circumference.

Expandable region 18 is shown in an expanded condition illustrated as 22 in FIG. 1B. Thus, in accordance with the principles illustrated in FIG. 1A and 1B, a cannula may have an anchoring member capable of being expanded for positioning or sealing at one or more selected longitudinal locations.

The expandable regions described above with respect to FIGS. 1A and 1B may be applied to the surface of cannula 10 or, in other embodiments, cannula 10 may be constructed with expandable regions or segments forming a portion of the wall of the cannula itself. In such an embodiment, the cannula may be made of flexible polymeric materials capable of binding to the expandable region.

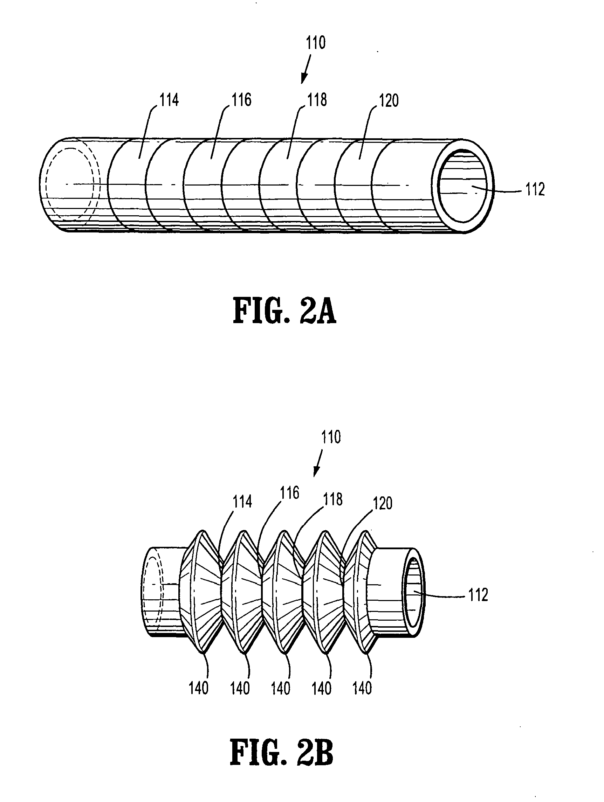

FIG. 2A illustrates a cannula 110 which possesses an anchoring member at one or more selected longitudinal locations. The cannula 110 possesses a longitudinally extending central working passage 112 and may, in embodiments, include a series of restrictive segments made of a shape memory material or electroactive polymer. The restrictive segments illustrated include segments 114, 116, 118, and 120. As an example, segments 114, 116, 118, and 120 may be restricted upon the application of heat where it is formed of a shape memory polymer, or upon the application of electricity from a low voltage electrical source (such as a battery), where the restrictive segment is formed of an electroactive polymer or a shape memory alloy such as Nitinol. The shape memory material or electroactive polymer utilized to form restrictive segments 114, 116, 118 and 120 may contract, resulting in cannula 110 adopting an accordion shape as depicted in FIG. 2B, with the formation of retention ribs 140 to assist retention of a cannula during a procedure. In other words, restrictive segments illustrated as segments 114, 116, 118, and 120 have a permanent shape like a ring with a first circumference and a temporary shape with a larger circumference.

In other embodiments, a cannula may possess segments thereon which undergo longitudinal contracting where the length of the cannula is shortened upon the application of energy. In embodiments, such a change may occur without altering the size of the orifice or diameter of the central passage of the cannula.

The restrictive segments described above in FIGS. 2A and 2B may be applied to the surface of cannula 110 or, in other embodiments, cannula 110 may be constructed with restrictive regions or segments forming portions of the wall of the cannula itself. Where the restrictive segments are applied to the surface of cannula, it may be advantageous for the cannula 110 to be constructed of a flexible polymeric material capable of being compressed.

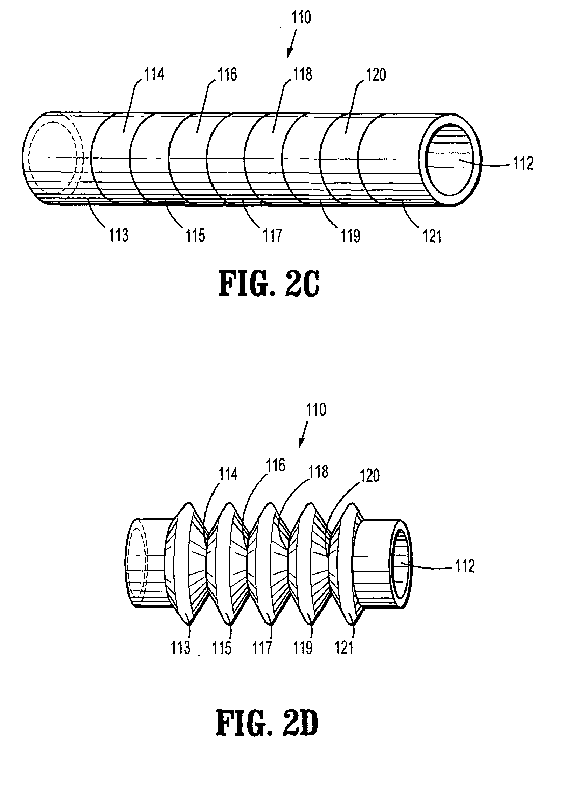

In other embodiments, as depicted in FIG. 2C, cannula 110 possessing longitudinally extending central working passage 112 may be made of shape memory materials having restrictive segments 114, 116, 118, and 120 therein which restrict upon application of energy, and also shape memory materials having expansive segments 113, 115, 117, 119, and 121 therein, which expand upon application of the same energy. Thus, as segments 114, 116, 118, and 120 restrict and segments 113, 115, 117, 119, and 121 expand, an accordion shape is thereby formed as depicted in FIG. 2D.

The expandable and restrictive segments described above in FIGS. 2C and 2D may be applied to the surface of cannula 110 or, in other embodiments, cannula 110 may be constructed with expandable regions or segments and restrictive regions or segments forming portions of the wall of the cannula itself.

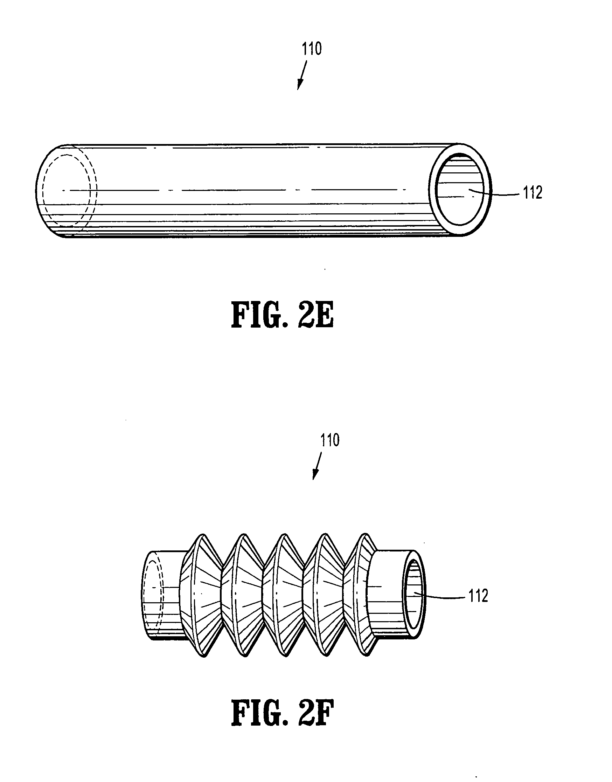

In alternate embodiments, cannula 110 possessing longitudinally extending central working passage 112 may be made of shape memory materials having a smooth, tubular temporary shape and an accordion permanent shape as depicted in FIGS. 2E and 2F. Upon application of sufficient energy, the cannula changes from its tubular temporary shape as depicted in FIG. 2E to its permanent accordion shape depicted in FIG. 2F.

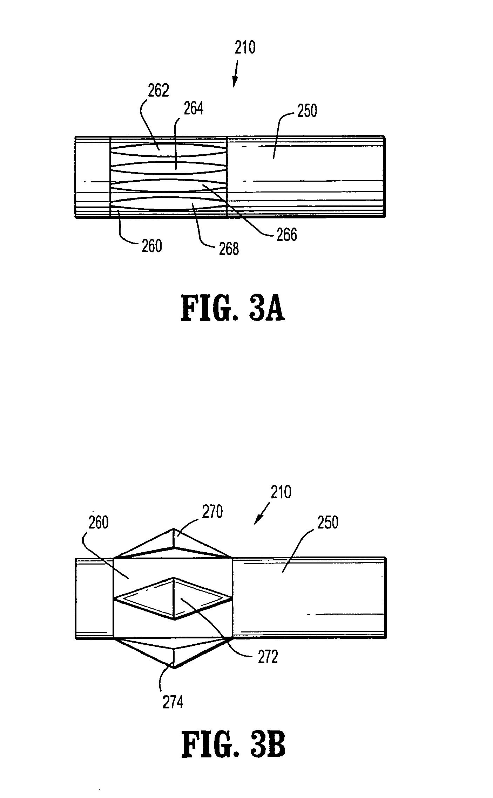

FIGS. 3A-3B illustrate a cannula 210, possessing a longitudinal shaft 250 with an expanding anchoring member 260 situated thereon. Anchoring member 260 may be made of a shape memory material or electroactive polymer. As depicted in FIG. 3A, anchoring member 260 may be placed along longitudinal shaft 250, with gaps 262, 264, 266 and 268 therein. There is no shape memory material or electroactive polymer in gaps 262, 264, 266 and 268; that is, anchoring member 260 is applied to longitudinal shaft 250 with gaps 262, 264, 266 and 268 which run longitudinally with the surface of longitudinal shaft 250. As is apparent from FIG. 3A, anchoring member 260 possesses as temporary shape having a smooth, flat configuration.

Upon application of suitable energy, such as heat or electricity, the shape memory material or electroactive polymer utilized to form anchoring member 260 may expand as depicted in FIG. 3B, with gaps 262, 264, 266 and 268 permitting such expansion and the formation of angular V shape protrusions 270, 272 and 274 from the surface of cannula 210 which may assist retention of cannula 210 during a procedure. Anchoring member 260 is shown in an expanded condition, which is its permanent shape, illustrated as angular V shape protrusions 270, 272 and 274 in FIG. 3B.

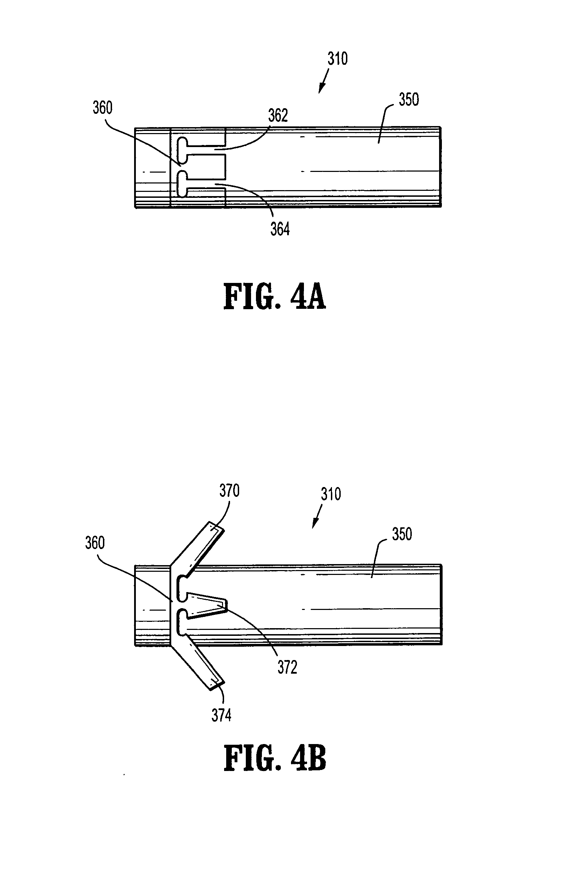

FIGS. 4A-4B illustrate a cannula 310, possessing longitudinal shaft 350 with an expanding anchoring member 360 situated thereon. Anchoring member 360 may be made of a shape memory material or electroactive polymer. As depicted in FIG. 4A, expanding anchoring member 360 may be placed along longitudinal shaft 350 in a configuration with gaps 362 and 364 therein. There is no shape memory material or electroactive polymer in gaps 362 and 364; that is, anchoring member 360 is applied to longitudinal shaft 350 with gaps 362 and 364 which run longitudinally with the surface of longitudinal shaft 350. As is apparent from FIG. 4A, anchoring member 360 possesses a temporary shape having a smooth, flat configuration.

Upon application of suitable energy, such as heat or electricity, the shape memory material or electroactive polymer utilized to form anchoring member 360 may expand to its permanent shape as depicted in FIG. 4B, with gaps 362 and 364 permitting such expansion and the formation of protrusions 370, 372 and 374 from the surface of cannula 310 which may assist retention of cannula 310 during a procedure. Anchoring member 360 is shown in an expanded condition illustrated as protrusions 370, 372 and 374 in FIG. 4B, thereby forming a frustoconical configuration.

Once a cannula has been secured to the site of an incision, in some embodiments an insufflation fluid or gas may by forced through the cannula and into the body cavity and surgical instruments and similar devices may be introduced through the cannula during a laparoscopic procedure.

In embodiments, it may be desirable to add additional components including medicinal agents with the shape memory polymers utilized to form the anchoring members of the present disclosure. The term "medicinal agent", as used herein, is used in its broadest sense and includes any substance or mixture of substances that have clinical use. Consequently, medicinal agents may or may not have pharmacological activity per se, e.g., a dye. Alternatively a medicinal agent could be any agent which provides a therapeutic or prophylactic effect, a compound that affects or participates in tissue growth, cell growth, cell differentiation, a compound that may be able to invoke a biological action such as an immune response, or could play any other role in one or more biological processes.

Examples of classes of medicinal agents which may be utilized in accordance with the present disclosure include antimicrobials; analgesics; anesthetics; antiinflammatory agents such as hormonal agents, hydrocortisone, prednisolone, prednisone, non-hormonal agents, allopurinol, indomethacin, phenylbutazone and the like; diagnostic agents; hemostats to halt or prevent bleeding; anticoagulants; antibiotics; anti-fungals; anti-virals; and immunological agents.

Suitable antimicrobial agents which may be included as a medicinal agent with the shape memory polymers utilized to form the anchoring members of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a medicinal agent with the shape memory polymers utilized to form the anchoring members of the present disclosure.

Examples of hemostat materials which can be employed include fibrin-based, collagen-based oxidized regenerated cellulose-based, and gelatin-based topical hemostats. Examples of commercially available hemostat materials include fibrinogenthrombin combination materials sold under the trade designations CoStasis^{™} by Tyco Healthcare Group, LP, and Tisseel^{™} sold by Baxter International, Inc. Hemostats herein also include astringents, for example, aluminum sulfate, and coagulants.

A single medicinal agent may be utilized with the shape memory materials utilized to form the anchoring member of a cannula of the present disclosure or, in alternate embodiments, any combination of medicinal agents may be utilized.

The medicinal agent may be disposed on a surface of the anchoring member, or impregnated in or combined with the shape memory materials utilized to form the anchoring member of a cannula of the present disclosure.

Cannulas of the present disclosure possessing an anchoring member made of shape memory materials or electroactive polymers may, in embodiments, avoid the need for extra deploying action, including that required with an anchoring balloon. The materials utilized to make the anchoring member are durable, the design is simple, and the cannula with the anchoring member of the present disclosure is simple to make utilizing existing materials and manufacturing processes. Thus, the present disclosure provides a cannula which is both economical and easy to use.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.

The present disclosure provides cannulas for use in surgery. The cannulas possess an anchoring member at a portion thereof which adopts an alternate shape upon the application of energy, thereby securing the cannula to tissue.

## Claims

1. A cannula comprising:
a cannula body possessing a longitudinally extending central working passage and at least one anchoring member, the anchoring member selected from the group consisting of shape memory polymers, shape memory metals, shape memory alloys, electroactive polymers, and combinations thereof.

2. The cannula of claim 1, wherein the anchoring member comprises a shape memory polymer selected from the group consisting of polyurethanes, poly(styrenebutadiene) block copolymers, polynorbomenes, caprolactones, dioxanones, diol esters, ether-ester diols, carbonates, and combinations thereof.

3. The cannula of claim 1, wherein the anchoring member comprises a shape memory polymer selected from the group consisting of oligo (epsilon caprolactone) diol, lactic acid, lactide, glycolic acid, glycolide, oligo (p-dioxanone) diol, trimethylene carbonate, and combinations thereof.

4. The cannula of claim 1, wherein the anchoring member comprises a shape memory polymer selected from the group consisting of poly(styrene-butadiene) copolymers, oligo (epsilon caprolactone) diol/oligo (p-dioxanone) diol copolymers, and poly(epsilon-caprolactone) dimethacrylate-poly (n-butyl acrylate) copolymers.

5. The cannula of claim 1, wherein the anchoring member comprises a shape memory polymer comprising a blend of materials selected from the group consisting of urethanes, lactic acid, glycolic acid, acrylates, caprolactones, homopolymers thereof, copolymers thereof, and combinations thereof.

6. The cannula of claim 1, wherein the anchoring member comprises a shape memory polymer which undergoes a change in shape at a temperature from about 30° C to about 50° C.

7. The cannula of claim 1, wherein the anchoring member comprises an electroactive polymer selected from the group consisting of poly(aniline), substituted poly(aniline)s, polycarbazoles, substituted polycarbazoles, polyindoles, poly(pyrrole)s, substituted poly(pyrrole)s, poly(thiophene)s, substituted poly(thiophene)s, poly(acetylene)s, poly(ethylene dioxythiophene)s, poly(ethylenedioxypyrrole)s, poly(p-phenylene vinylene)s, and combinations thereof.

8. The cannula of claim 7, wherein the electroactive polymer undergoes a change in shape upon the application of electricity in amounts from about 5 volts to about 30 volts.

9. The cannula of claim 1, wherein the anchoring member is secured to the shaft by at least one of chemical methods, physical methods, and combinations thereof.

10. The cannula of claim 9, wherein the anchoring member is secured to the shaft chemically by one of adhesives, sealants, and glues.

11. The cannula of claim 9, wherein the anchoring member is secured to the shaft physically by locking rings located on the proximal end and distal end of the anchoring member.

12. The cannula of claim 1, wherein the anchoring member has a permanent shape comprising a ring with a first expanded annular diameter and a temporary shape with a compressed annular diameter.

13. The cannula of claim 1, wherein the anchoring member has a permanent shape comprising an expanded annular diameter of from about 0.75 inches to about 1.75 inches and a temporary shape comprising a compressed annular diameter of from about 0.002 inches to about 0.01 inches.

14. The cannula of claim 1, wherein the anchoring member has a permanent shape comprising a ring with a first compressed annular diameter and a temporary shape with a larger expanded annular diameter.

15. The cannula of claim 1, wherein the anchoring member has a permanent shape comprising a compressed diameter of from about 0.002 inches to about 0.01 inches and a temporary shape comprising an expanded diameter of from about 0.75 inches to about 1.75 inches.

16. The cannula of claim 1, wherein the anchoring member has a permanent shape comprising angular protrusions and the temporary shape comprises a smooth, flat configuration.

17. The cannula of claim 16, wherein the cannula adopts an accordion shape upon application of energy.

18. The cannula of claim 17, wherein the anchoring member includes at least one restrictive segment which restricts upon application of energy.

19. The cannula of claim 16, wherein the anchoring member has a permanent shape comprising angula V shape protrusions and the temporary shape comprises a smooth, flat configuration, or wherein the anchoring member has a permanent shape comprising a frustoconical configuration and the temporary shape comprises a smooth, flat configuration.

20. The cannula of claim 1, wherein the anchoring member is applied to a surface of the cannula, or wherein at least a portion of a wall of the cannula wall comprises the anchoring member or wherein the anchoring member further comprises a medicinal agent.
